(19) **Europäisches Patentamt**
**European Patent Office**
**Office européen des brevets**

(11) **EP 2 374 912 A1**

(12) **EUROPEAN PATENT APPLICATION**
published in accordance with Art. 153(4) EPC

(43) Date of publication:
**12.10.2011 Bulletin 2011/41**

(51) Int Cl.:
*C23C 4/06* (2006.01)     *C23C 4/04* (2006.01)

(21) Application number: **08878848.4**

(22) Date of filing: **17.12.2008**

(86) International application number:
**PCT/CN2008/073544**

(87) International publication number:
**WO 2010/069104 (24.06.2010 Gazette 2010/25)**

(84) Designated Contracting States:
**AT BE BG CH CY CZ DE DK EE ES FI FR GB GR HR HU IE IS IT LI LT LU LV MC MT NL NO PL PT RO SE SI SK TR**

(71) Applicant: **Master Technology Company Limited Cheung Sha Wan, KL, Hong Kong (CN)**

(72) Inventor: **Master Technology Company Limited Cheung Sha Wan, KL, Hong Kong (CN)**

(74) Representative: **Brand, Thomas Louis**
**W.P. Thompson & Co.**
**55 Drury Lane**
**London WC2B 5SQ (GB)**

(54) **ANTIBACTERIAL COATING, ITS PREPARATION METHODS AND METALWORK CONTAINING THE COATING**

(57)     Disclosed are an antimicrobial coating and a product comprising the same. Also disclosed are processes for preparing the antimicrobial coating and the metal product comprising the coating.

EP 2 374 912 A1

**Description**

TECHNICAL FIELD

The present application is generally directed to an antimicrobial coating and a product comprising the coating. The present application is also directed to a process for preparing an antimicrobial coating and a metal product comprising the coating.

BACKGROUND

The antimicrobial metals in the prior art mainly resides in: (1) the existence in the form of an alloy, i.e. the antimicrobial metals are cast with other metals to form an alloy; and (2) independently coated on the surface of a metal or non-metal to form an antimicrobial metallic coating.

Patent application WO 99/64640 A1 to Yokota Takeshi, et al discloses a stainless steel having antimicrobial properties. When continuous cast having different continuous casting rates is performed, a stainless steel having different chemical components is prepared by a steel-making technology, in which chrome is more than 10% by weight, silver is 0.001-0.30% by weight and silver oxide is more than 0.0005% by weight.

Patent application WO 99/47721 A1 to Tochihara Misako, et al discloses an antimicrobial stainless steel. A stainless steel having different chemical components is obtained by smelting, in which chrome is more than 10% by weight and silver is in 0.0001-1% by weight. This steel comprises more than 0.001% total area ratio of at least one of the silver particles, silver oxide and silver sulfide.

Patent application CN 1687200 A Dehuan HUANG, et al discloses a process for preparing antimicrobial plastics with inorganic nano-silver loaded powders. Among other things, the modifier is used to modify nano-silver powders in which inorganic superfine powders are carriers, and then the modified silver powders are spin-coated on the processed surface of the plastics. The effects of uniformly dispersing and keeping the initial mechanical properties of the plastics can be achieved.

Patent application WO 99/25898 A1 to Shigeru Keijiro, et al discloses a process for preparing an antimicrobial metal product. The process comprises coating a suspension or solution of antimicrobial component particles on the surface of a metal product and pressing the obtained coated surface of the metal product without heating.

However, the processes as previously described have several disadvantages:

(1) The process for manufacturing an alloy product prepared by adding an antimicrobial metal into silver or copper is quite complicated and difficult. Furthermore, since the content of silver or copper in the whole metal cannot be too small, the cost of the product is very high.

(2) Originally, coating an antimicrobial metal on a substrate to form a coating film might meet the production and cost requirements, because the antimicrobial metal to be applied can be gathered on the surface of the substrate while the substrate does not comprises any antimicrobial substance. With regard to the substrate, it is not necessarily a metal, it can be fabrics, papers, woods, glass, plastics, ceramics, ant the like. The use of the above product as a substrate is quite extensive. However, the defects of the product reside in that the adhesive (if used) and the antimicrobial metal are not wear-resistant, impact-resistant. Moreover, all the used adhesives are not resistant to high temperature ($\leq$ 260 °C).

SUMMARY

The present application is intended to solve one of the disadvantages in the prior art, and therefore provides an antimicrobial coating, a producting comprising the coating and the process for preparing the same.

Therefore, in an aspect, the present application is directed to an antimicrobial coating, comprising an antimicrobial effective amount of silver powders and stainless steel powders, wherein the sliver powders and the stainless steel powders are present in the form of physical doping in the antimicrobial coating.

In other aspect, the present application is also directed to a metal product, wherein the surface of a substrate is coated with an antimicrobial coating comprising an antimicrobial effective amount of silver powders and stainless steel powders, wherein the silver powders and the stainless steel powders are present in the form of physical doping in the antimicrobial coating.

In still another aspect, the present application is directed to a process for preparing an antimicrobial coating, comprising:

(1) mixing an antimicrobial effective amount of silver powders and stainless steel powders; and
(2) spraying the mixture of the silver powders and stainless steel powders on a surface to be processed,

wherein the silver powders and the stainless steel powders are present in the form of physical doping in the antimicrobial coating.

In yet another aspect, the present application is directed to an antimicrobial coating prepared with the following process comprising:

(1) mixing an antimicrobial effective amount of silver powders and stainless steel powders; and
(2) spraying the mixture of the silver powders and the stainless steel powders on a surface to be processed,

wherein the silver powders and stainless steel powders are present in the form of physical doping in the antimicrobial coating.

In still another aspect, the present application is directed to a process for protecting against microbes, comprising applying an antimicrobial coating comprising an antimicrobial effective amount of silver powders and stainless steel powders to a product in need of antimicrobial action, wherein the silver powders and stainless steel powders are present in the form of physical doping in the antimicrobial coating.

In some embodiments, the coating as provided can effectively coat the antimicrobial metal on the surface of a metal product, and the coating is resistant to high temperature, wear and impact.

BRIEF DESCRIPTION OF DRAWINGS

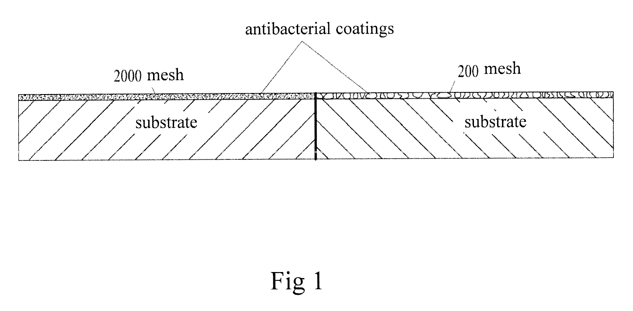

Fig. 1 is a schematic diagram of a substrate coated with the antimicrobial coating (200 and 2000 mesh) of an embodiment in the present application.

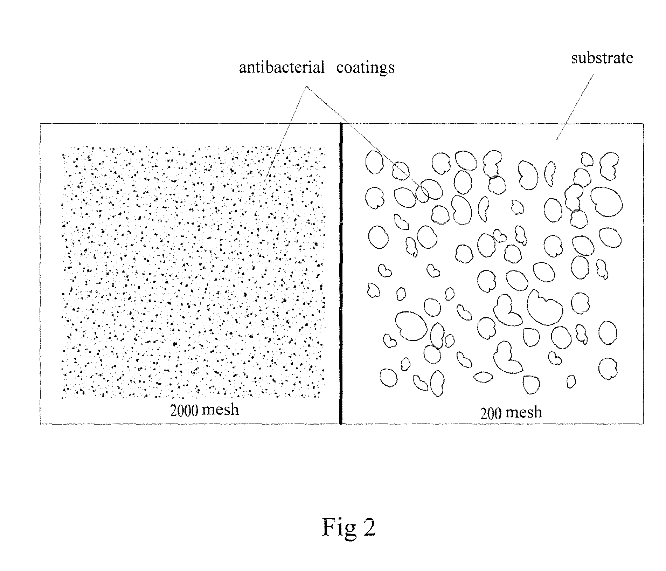

Fig. 2 is a schematic diagram of an antimicrobial metallic coating (200 and 2000 mesh) in an embodiment.
Fig. 3 shows a test piece inoculated with bacteria solutions and coated with a film on a plate.

DETAILED DESCRIPTION

In the following description, certain specific details are included to provide a thorough understanding of various disclosed embodiments. One skilled in the relevant art, however, will recognize that the embodiments may be practiced without one or more of these specific details, or with other methods, components, materials, etc.

Unless the context requires otherwise, throughout the specification and claims which follows, the term "comprise" and variations thereof, such as "comprises" and "comprising" are to be construed in an open, inclusive sense, which is as "include, but not limited to".

Reference throughout this specification to "one embodiment", or "an embodiment", or "in another embodiment", or "some embodiments", or "in some embodiments" means that a particular referent feature, structure or characteristic described in connection with the embodiments is included in at least one embodiment. Therefore, the appearance of the phases "in one embodiment", or "in an embodiment", or "in another embodiment", or "in some embodiments" in various places throughout this specification are not necessarily all referring to the same embodiment. Moreover, the particular features, structures or characteristics may be combined in any suitable manner in one or more embodiments.

It should be noted that, as used in this specification and the appended claims, the singular forms "a", "an" and "the" include plural referents unless the content clearly indicate otherwise. Therefore, for example, "mixing an antimicrobial metal with a stainless steel" comprises mixing one antimicrobial metal (such as silver) with one stainless steel (such as nickel-based containing chromium stainless steel), mixing one antimicrobial metal (such as copper) with several stainless steels (such as nickel-based containing chromium stainless steels and cobalt-based containing chromium stainless steels), mixing several antimicrobial metals (such as silver and copper) with one stainless steel (such as nickel-based containing chromium stainless steel), and mixing several antimicrobial metals (such as silver and copper) with several stainless steels (such as nickel-based containing chromium stainless steel and cobalt-based containing chromium stainless steel). It should be also noted that the use of "or" means "and/or" unless stated otherwise.

DEFINITIONS

As used in specification and appended claims, unless specified to the contrary, the following terms have the meaning indicated:

The term "antimicrobial" or "antimicrobial action" as used herein refers to the general term of bacteriostasis and bactericidal action. The term "bacteriostasis" or "bacteriostatic action" refers to an action of inhibiting the growth and reproduction of microbes. The term "bactericidal" or "bactericidal action" refers to an action of killing trophosomes and propagules of microbes.

The term "physical doping" as used herein refers to that after an antimicrobial coating is formed with an antimicrobial metal and other metals (such as stainless steel), the antimicrobial metal particles and other metal particles fill with each other in the interstices of the surface of a substrate so as to form a coating doped with physical particles, i.e. the antimicrobial particles and the metal particles can be distinguished in the coating.

The term "antimicrobial effective amount" as used herein refers to an antimicrobial amount which can achieve the desired antimicrobial action. Generally, the antimicrobial effects as needed to be achieved may be different according to different antimicrobial requirements. In some embodiments of the present application, for example, an antimicrobial effective amount of silver powders may refer to about 0.01-30% of antimicrobial metallic silver powders. In some other embodiments, an antimicrobial effective amount of silver powders may refer to about 1-20% of antimicrobial metallic silver powders. In some other embodiments, an antimicrobial effective amount of silver powders may refer to about 5-10% of antimicrobial metallic silver powders.

The term "antimicrobial metal" as used herein refers to a metal which can play a role in an antimicrobial action. The conventional antimicrobial metals in the prior art include, but are not limited to, silver, copper, zinc, cobalt, nickel, and the like.

The term "metal" as used herein should be understood in a broader sense, i.e., it comprises a single metal, or a mixture of various metals, as well as various alloys, such as an aluminum alloy and a stainless steel.

The term "stainless steel" as used herein refers to the general term of an alloy steel resistant to air, acid, base, salt, and the like corrosion. There are various stainless steels. In accordance with the structure of metallographic phase, the stainless steels comprise martensitic, austenitic, ferrite, and duplex type stainless steels. In accordance with the chemical components, the stainless steels comprise two major systems of chromium stainless steel and nickel-chromium stainless steel represented by Cr13 and Cr18Ni8, respectively. Other stainless steels are developed on the basis of the two stainless steels. In accordance with the environmental media during use, the stainless steels comprise stainless steels resistant to nitric acid, sulfuric acid, urea and seawater, and the like. In accordance with the corrosion resistance, the stainless steels comprise anti-pitting stainless steel, stress corrosion resistance stainless steel, abrasion resistance stainless steel, and the like. In accordance with functional characteristics, the stainless steels comprise non-magnetic stainless steel, free cutting stainless steel, high strength stainless steel, low temperature and super low temperature stainless steel, superplastic stainless steel, and the like.

The term "austenite" as used herein refers to an interstitial solid solution, in which carbon dissolves in $\gamma$-Fe, having the face-centered cubic structure and no magnetism. Austenite is a tissue of a conventional steel at a high temperature and exists in a certain range of temperatures and components. Some quenched steels can retain some austenites at the ambient temperature. Such an austenite is called as residue austenite. In addition to carbon in an alloy steel, other alloy elements can also dissolve in an austenite, and enlarge or reduce the temperature and component range of the austenitic stable region. For example, the austenitic critical converting temperature can be lowered below the ambient temperature by adding manganese and nickel, which makes the steel to remain the austenitic tissue at ambient temperature, i.e. the so-called austenitic steel.

The term "austenite type stainless steel" as used herein refers to a stainless steel having austenitic tissues at the ambient temperature. When comprising about 18% Cr, 8%-10% Ni, about 0.1% C, the steel has stable austenitic tissues. The austenitic chromium-nickel stainless steels comprise the well-known Cr18Ni8 steels and high Cr-Ni serial steels which are developed by increasing the contents of Cr and Ni and adding elements such as Mo, Cu, Si, Nb, Ti, and the like on the basis of Cr18Ni8 steels. The austenitic stainless steels are non-magnetic and have high toughness and plasticity. However, the strength of the austenitic stainless steels is lower and cannot to be strengthened by phase transition, while can be only strengthened by cold processing. If elements, such as S, Ca, Se, Te and the like, are added, the austenitic stainless steels have good free-cutting machinability. In addition to resistance to corrosion of oxidative acidic media, such steels also have resistance to corrosion of sulfuric acid, phosphoric acid, formic acid, acetic acid, urea and the like, were comprising elements such as Mo, Cu, and the like. If such steels comprise carbon content lower than 0.03% or Ti, Ni, the resistance to intergranular corrosion significantly increases. Austenitic stainless steels with high silicon content have good corrosion resistance to concentrated nitric acid.

The examples of austenite type stainless steels comprise, but are not limited to, 201, 202, 205, 301, 302, 302B, 303, 303Se, 304, 304L, 302HQ, 304N, 305, 308, 309 type stainless steels.

The term "martensite" as used herein refers to a supersaturated solid solution, in which g carbon dissolves in $\alpha$-Fe, having body-centered cubic structure and ferromagnetism and higher resistivity. A martensite is a metastable tissue of single-phase. The shape of a martensite generally is strip, lens sheet or both. A martensite has higher hardness and strength. A martensite with strip shape has better toughness while a martensite with lens sheet shape has poor toughness. After a steel is austenitized, a product of martensitic transformation can be obtained by cooling at a certain higher rate at a lower temperature. Of various tissues of steels, the specific volume of martensite is the highest. Generally, the more carbon content in steels is, the higher hardness of martensite will be. Obtaining a martensite by quenching is an important means to strengthen steels. Maraging steels having high strength and toughness can be prepared by adding suitable amount of alloy elements such as cobalt, molybdenum, titanium and the like in a ultra-low carbon iron-nickel alloy.

The term "martensite type stainless steel" as used herein refers to the stainless steels having martensitic microscopic tissues during use. The chromium content in the martensite type stainless steels is 13-18%. After quenching and tempering, the martensite type stainless steels can be used in steam turbine blades (containing lower carbon content), medical surgical instruments, measuring tools, springs (containing higher carbon content) and the like.

The examples of martensite type stainless steels include, but are not limited to, 403, 410, 414, 416, 416Se, 420, 431, 440A, 440B, 440C type stainless steels.

The term "ferrite" as used herein refers to α-Fe (or β-Fe) and a solid solution on the basis of the same. A ferrite has body-centered cubic lattice. A ferrite is formed by the eutectoid precipitation of an austenite having hypoeutectoid components. This portion of ferrite is referred as proeutectoid ferrite or ferrite with free tissues. According to different forming conditions, the proeutectoid ferrites have different shapes, such as equiaxial, intergranular, spindle, serrated, acicular shapes and the like. The ferrite is also the matrix of pearlitic tissues. The ferrites are main constituent phases in the hot-rolling (normalizing) and anneal tissues of carbon steels and low-alloy steels. The components and tissues of ferrites have significant impacts on the technical properties of steels and the use performances of steels in some situations.

The term "ferrite type stainless steel" as used herein refers to a stainless steel of which the tissues are mainly ferrites during use. The chromium content in ferrite type stainless steels is generally 12-230%. Ferrite type stainless steels have body-centered cubic structure. Such steels generally comprise no nickel, but sometimes comprise a small amount of elements such as Mo, Ti, Nb and the like. Such steels have advantages such as high thermal conductivity coefficient, low coefficient of expansion, good oxidation resistance, excellent stress corrosion resistance and the like, and therefore are usually used to prepare parts resistant to air, stream, water and oxidizing acids corrosions.

The examples of ferrite type stainless steels include, but are not limited to, 405, 409, 429, 430, 430F type stainless steels.

The term "duplex type stainless steel" as used herein refers to a stainless steel having about 50% of ferrite and about 50% of austenite. The content of the less phase in a duplex type stainless steel is generally required to reach at least 30%. The key characteristic of the duplex type stainless steel resides in that the yield stress can reach up to 400-550 MPa, which is twice of the conventional stainless steel. Therefore, the duplex type stainless steels can save materials and reduce the manufacturing costs of devices. With regard to the corrosion resistance, especially under the condition of worse environmental media (such as seawater, higher chloride ion content), the properties of duplex type stainless steels such as resistance to pitting corrosion, crevice corrosion, stress corrosion, and corrosion fatigue are significantly better than those of common austenite stainless steels and can be on a par with high-alloy austenite stainless steels. The duplex type stainless steels have good welding performances. Comparing with ferrite stainless steels and austenite stainless steels, the heat-affected zone of the duplex type stainless steels are neither the same as that of ferrite stainless steels, in whic the toughness is greatly reduced as the grains are seriously coarsened, nor as that of austenite stainless steels, which is more susceptible to welding heating cracks.

The examples of duplex stainless steels include, but are not limited to, 329 and 2205 type stainless steels.

The term "elements beneficial to the human body" as used herein refers to elements which cannot be synthesized by the human body itself and must be ingested outside. If lacking one or more such elements, the human body will suffer from various diseases. Element chromium involves in metabolism of sugar and fat, increases the decomposition and excretion of cholesterol and reduces the incidence of coronary disease. Nickel is a component constituting cells and can activate insulins and reduce the content of blood sugar. Manganese involves in the growth and development of bones and the hematopoiesis process, and closely relates to the reproductive function. Manganese is a component of various enzymes and directly involves in metabolism of the human body. Cobalt is an active center of vitamin B12, and can facilitate the erythropoiesis and affect the absorption and metabolism of phosphorus, magnesium, iron. Nutritionist indicates that the incidence of diabetes and cardiovascular diseases increases if the meals lack trace elements, such as chromium, nickel, molybdenum, cobalt and the like, for a long time.

**Antimicrobial Mechanism**

In general, antimicrobial metal ions can dissolve from an antimicrobial coating. When contacting with bacteria, the antimicrobial metal ions dissolved from an antimicrobial coating (such as silver ions, copper ions and the like) damage cell walls of bacteria or enter the bacteria through the cell walls to damage the conducting tissues, prevent the metabolism of the cells so as to kill the bacteria and achieve the purpose of sterilization. Moreover, antimicrobial metallic ions, such as silver and the like, have certain catalytic effects and can convert the water and oxygen adsorbed on the surface of the coating into hydroxyl groups and active oxygen. The active oxygen has strong oxidation capacity and damages the cell walls of the bacteria so as to kill the bacteria.

**Antimicrobial Performance Test Method and Antimicrobial Effects**

Antimicrobial Effects

The antimicrobial effects of a product processed with an antimicrobial method can be obtained by the value of antimicrobial activity. The value should not be less than 99%.

Antimicrobial Performance Test Method

The test method for plastic products is used. The test method is suitable for products such as plastic products, metal products and ceramic products except for fiber products.

1. Bacteria Used in Tests.

> (1) *Escherichia coli*
> (2) *Staphylococcus aureus*
> (3) *Pseudomonas aeruginosa*
> (4) *Klebsiella pneumonia*
> (5) *Salmonella aertrycke*

**Table 1** shows the representative bacterial stains, the stains deposit numbers and the stains depository institutions.

**Table 1** Bacterial Stains Used in Tests

| Bacterial Species | Stains Deposit Nos. | Stains Depository Institutions |
|---|---|---|
| *Escherichia coli* | ATCC 25922 | American Type Culture Collection |
| *Staphylococcus aureus* | ATCC 6538 | American Type Culture Collection |
| *Pseudomonas aeruginosa* | ATCC 9027 | American Type Culture Collection |
| *Klebsiella pneumoniae* | ATCC 10031 | American Type Culture Collection |
| *Salmonella aertrycke* | NICPBP 50115 | National Institutes for the Control of Pharmaceutical and Biological Products |

2. Reagents, Materials, Apparatuses and Devices

| | |
|---|---|
| Alcohol | Microbial grade |
| Beef extract | Microbial grade |
| Peptone | Microbial grade |
| Sodium chloride (NaCl) | Microbial grade |
| Purified water | Microbial grade |
| Agar culture medium | Microbial grade |
| Yeast extract | Microbial grade |
| Glucose | Microbial grade |
| Casein peptone | Microbial grade |
| Soybean peptone | Microbial grade |
| Lecithin | Microbial grade |
| Nonionic surfactant-tweenum 80 | Microbial grade |
| Potassium dihydrogen phosphate ($KH_2PO_4$) | Microbial grade |
| Dipotassium hydrogen phosphate ($K_2HPO_4$) | Microbial grade |
| Sodium hydroxide (NaOH) | Microbial grade |

(continued)

| | |
|---|---|
| Hydrochloric acid (HCl) | Microbial grade |
| Cotton plug* | OME cotton |
| Platinum inoculating ring | With a ring of about 4 mm on the top |
| Dry heat Sterilizer | Microbial grade |
| Pressure steam sterilizer | Microbial grade |
| Security console | Microbial grade |
| Clean workbench | Microbial grade |
| Straw | Microbial grade |
| Constant temperature incubator | Microbial grade |
| Culture dish | Microbial grade |
| Stomacher | Microbial grade |
| Film | Microbial grade |

\* the silicone, metal or morton plug can be used as well

3. Sterilization Method

(1) Dry Heat Sterilization

Articles needed to be sterilized are placed in a dry heat sterilizer at a temperature of 160-180 °C and kept 30-60 minutes. Note that after dry heat sterilization, the related devices cannot be used if the cotton plug or package is wet.

(2) High pressure stream sterilization

Water is added into a pressure stream sterilizer. Articles needed to be sterilized are placed in the metal net basket on the metal grid of the pressure stream sterilizer. Then the pressure stream sterilizer is tightly capped. The articles are heated for 15-20 minutes under the pressure of 103000 MPa at the temperature of 121 °C. After the heating is finished, the pressure stream sterilizer is naturally cooled below 100 °C. The exhaust valve is opened to exhaust the gas, and then the cap is opened. The sterilized articles are taken out. If necessary, the pressure stream sterilizer can be cooled on a clean workbench. The pressure stream sterilizer should keep clean and avoid contaminating cultures or reagents during the process. If necessary, the pot can be washed with neutral detergents, and flushed with sufficient water.

(3) Flame sterilization

The reagent is put in flame of air or alcohol for sterilization. When a platinum ring is used, it is heated until red. When a test tube is used, it is contacted with flame for 2-3 seconds.

(4) Apparatus sterilization

A basic or neutral detergent is used to wash glass wares such as test tubes or beakers. The glass wares are then fully washed with water and dried. The glass wares can be used after being sterilized in a dry heat sterilizer or a high pressure stream sterilizer.

4. Bacteria culture medium

The following bacteria culture media are used. Commercially available bacteria culture media which has the same composition can also be used.

(1) Nutrient broth (NB)

5.0 g beef extract, 10.0 g peptone and 5.0 g sodium chloride are added to 1000 mL purified water or deionized water. The mixture is placed in a flask to mix and dissolve. 0.1 mol/L NaOH solution or hydrochloric acid solution is used to adjust pH to 7.0-7.2 (25 °C). The resultant solution is then sterilized with a high pressure stream sterilizer for 30 minutes at 121 °C. If not used immediately after preparation, the solution should be preserved at 5-10 °C for less than one month.

(2) Nutrient agar culture medium (NA)

5.0 g beef extract, 10.0 g peptone, 5.0 g sodium chloride and 15.0 g agar are added to 1000 mL purified water or deionized water. The mixture is placed in a flask to mix and then is heated in a boiling bath to sufficiently dissolve. 0.1 mol/L NaOH solution or hydrochloric acid solution is used to adjust pH to 7.0-7.2 (25 °C). The resultant solution is then sterilized with a high pressure stream sterilizer for 30 minutes at 121 °C. If not used immediately after preparation, the solution should be preserved at 5-10 °C for less than one month.

(3) Standard agar culture medium

2.5 g Yeast extract, 5.0 g peptone, 1.0 g glucose and 15.0 g agar are added to 1000 mL purified water or deionized water. The mixture is placed in a flask to mix and then is heated in a boiling bath to sufficiently dissolve. 0,1 mol/L NaOH solution or hydrochloric acid solution is used to adjust pH to 7.0-7.2 (25 °C). The resultant solution is then sterilized with a high pressure stream sterilizer for 30 minutes at 121 °C. If not used immediately after preparation, the solution should be preserved at 5-10 °C for less than one month.

(4) Sloped culture medium

6-10 mL nutrient agar culture (NA) which is melted after heating is poured into a test tube. A high pressure stream sterilizer is used to sterilize the nutrient agar culture and the tube for 30 minutes at 121 °C. After sterilization, the test tube is placed on a clean workbench by inclining 15° and then the nutrient agar culture solidifies. If not used immediately after preparation, the sloped culture medium should be preserved at 5-10 °C for less than one month.

(5) SCDLP broth culture medium

17.0 g casein peptone, 3.0 g soybean peptone, 5.0 g sodium chloride, 2.5 g potassium hydrogen phosphate, 2.5 g glucose and 10.0 g lecithin are added to 1000 mL purified water or deionized water. The mixture is placed in a flask to mix and sufficiently dissolve. 7.0 g nonionic surfactant is added. 0.1 mol/L NaOH solution or hydrochloric acid solution is used to adjust pH to 6.8-7.2 (25 °C). When the culture medium is used, the culture medium is added in a test tube or an erlenmeyer flask. A high pressure stream sterilizer is used to sterilize the culture medium for 30 minutes at 121 °C. If not used immediately after preparation, the culture medium should be preserved at 5-10 °C for less than one month.

(6) Phosphoric acid buffer

To a flask is added 34.0 g potassium dihydrogen phosphate. 500 mL purified water or deionized water is added to the flask to sufficiently dissolve potassium dihydrogen phosphate. 0.1 mol/L NaOH solution is used to adjust pH to 6.8-7.2 (25 °C). Purified water or deionized water is then added up to 1000 mL. When the solution is used, the solution is added in test tubes or erlenmeyer flasks, and sterilized with high pressure stream sterilizer for 30 minutes at 121 °C. The phosphoric acid buffer which is preserved for one or more months after preparation can not be used.

(7) Phosphoric acid buffering physiological saline

Physiological saline (comprising 0.85% sodium chloride) is used to dilute the above phosphoric acid buffer (800 times dilution). When phosphoric acid buffering physiological saline is used, it is added in a test tube or an erlenmeyer flask. A high pressure stream sterilizer is used to sterilize phosphoric acid buffering physiological saline for 30 minutes at 121 °C. The phosphoric acid buffer which is preserved for one or more months after preparation cannot be used.

5. Preservation of bacteria

The transfer of bacteria should be conducted sterilely. The platinum inoculating ring sterilized by flame is used to scrape a ring of preserved bacteria, and then streak-inoculated to a sloped culture. After culturing for 24-48 hours at 37 °C ± 1 °C, the bacteria are preserved at 5-10 °C. The transfer should be conducted once again within a month. However, the generation number of the switch is limited within 10 generations and the bacteria switch cultured is preserved for less than one month.

6. Test steps

(1) Pre-culturing test bacteria

The above preserved stains are inoculated to a sloped culture with a platinum inoculating ring. The stains are cultured for 16-24 hours at 35 °C $\pm$ 1 °C, and then transferred again, and cultured for 16-20 hours.

(2) Preparing test pieces

A square piece with 50 mm $\pm$ 2 mm (the thickness is less than 10 mm) is cut from the flat portion of a product as a test piece with a standard size. Six crude test pieces and three antimicrobial test pieces are prepared. Of the six crude test pieces, the viable counts of three pieces are immediately measured after inoculating, while the viable counts of the other three pieces are measured after inoculating for 24 hours.

(3) Washing of test pieces

The above test pieces are slightly wiped two to three times with absorbent cottons stained with alcohol and then dried sufficiently. If such treatment affects the test results, other suitable methods can be used to clean the test pieces. Non-cleaned test pieces can be used.

(4) Preparing test bacteria solutions

The above broth culture (NB) is diluted 500 times with purified water. 0.1 mol/L of NaOH solution or hydrochloric acid solution is used to adjust pH to 6.8-7.2 (25 °C). The 1/500 NB inoculating solution is prepared by high pressure stream sterilization. One inoculating ring of the above pre-cultured bacteria is taken and dissolved in 1/500 NB inoculating solution. The resultant solution is diluted to $2.5 \times 10^3$ -$10 \times 10^3$/mL bacterial counts. If not used immediately, the prepared bacteria solution should be preserved at 0 °C and used within 4 hours.

(5) Inoculating

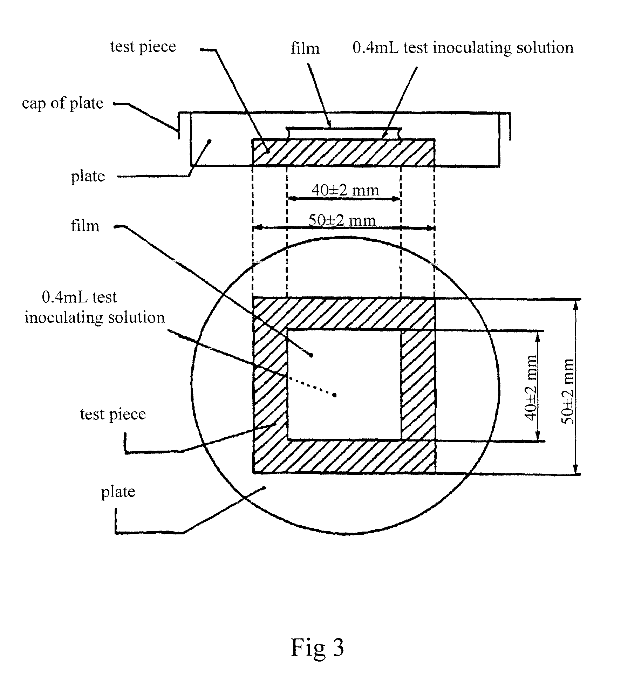

The washed test pieces are placed on sterilized plates, respectively. The test surface is placed upwards. 0.4 mL test bacteria solution is accurately taken with a pipette and dropped on each test piece in a plate. The test piece is covered with film. The film is pressed carefully such that the test bacteria solution spreads. It should be noted, that the inoculating solution shall not overflow from the outer edge of the film. Finally, a cap is put on the plate (see Fig 3).

It should be noted that the test surface is the surface of the antimicrobial product. The standard size of the film should be a square with 40 mm $\pm$ 2 mm.

(6) Culturing

The plates with the test pieces inoculated with the test bacteria solutions (three crude test pieces and three antimicrobial product test pieces) are cultured for 24 $\pm$ 1 hours at 35 °C $\pm$ 1 °C under the relative humidity of not less than 90%.

(7) Eluting

a) Test pieces inoculated with test bacteria solutions

After inoculating the three crude test pieces, the cover films and test pieces are immediately placed in a stomacher sack with tweezers. 10 mL SCDLP broth culture fluid is added with a pipette. The test pieces and cover films in the sack are rubbed with hand for eluting. The viable counts in the eluate are calculated.

b) Cultured test pieces

The above cultured test pieces are eluted in the same manner as that for the crude test pieces. The viable counts in the eluate are immediately calculated.

(8) Plate viable bacteria count method

1mL the above eluate is accurately drew with a pipette and put into a test tube containing 9.0 mL physiological saline phosphoric acid buffer to sufficiently mix. 1 mL the resultant solution is drew from the test tube with a new pipette and put into another test tube containing 9.0 mL physiological saline phosphoric acid buffer to sufficiently mix. The procedure is repeated to conduct a series of 10-fold increasing dilutions. 1 mL eluate and each dilution are added into two sterilized plates, respectively. 15-20 mL standard agar culture medium which is heated to 46-48 °C is placed on each plate to sufficiently mix. The plate is covered with a cap and placed at the ambient temperature. After the culture medium solidifies, the plate is turned over and cultured for 40-48 hours at 35 °C $\pm$ 1 °C. After culturing, the number of the plates in which the dilution has 30-300 bacterial counts is calculated. If the bacterial counts on the plate with 1 mL eluate are less than 30, the bacteria counts of the plate are calculated. If no bacteria counts are on the plate, it is recorded as "< 1".

7. Calculation of viable counts

Viable counts are calculated with the bacterial counts according to the following formula:

$$N= C \times D \times V$$

wherein

N: viable counts (each test piece)
C: bacterial counts (the mean value of the bacterial counts of the two plates)
D: dilution rate (the rate by which the dilution is made up to the plate)
V: volume (mL) of the SCDLP broth culture medium used in elution

The viable counts are recorded with two significant digits after rounding up or down the third significant digit. If the bacterial counts are "< 1", the viable counts are recorded as "< 10" (under the condition of 10 mL). After the mean value of the viable counts is calculated, the arithmetic mean value of viable counts in each of the three test pieces is calculated and recorded with two significant digits after rounding up or down the third significant digit. If the mean value of the viable counts is "< 10", the mean value of the viable counts can be recorded as 10.

8. Test results

(1) Judging conditions for test establishment
If the following three conditions are met, the test is determined to be valid. Unless all the conditions are met, the test will be determined to be invalid and shall be carried out again.

a) The directly calculated logarithm value of viable counts obtained from the crude test piece after inoculating should meet:

$$(L_{max} - L_{min})/L_{mv} \leq 0.2$$

wherein

$L_{max}$: maximum logarithm value of viable counts
$L_{min}$: minimum logarithm value of viable counts
$L_{mv}$: logarithm value of the mean viable counts

b) The directly calculated logarithm value of viable count obtained from the crude test piece after inoculating should be in the arrange of $1.0 \times 10^5$ -$4.0 \times 10^5$ cfu/piece.
c) The viable counts of all the three test pieces obtained from the crude test pieces after culturing for 24 hours should not be less than $1.0 \times 10^3$ cfu/piece. However, when cover films are used to cover the crude test pieces, the viable counts of all the three test pieces after placing for 24 hours should not be less than $1.0 \times 10^4$ cfu/piece.

(2) If the test is established, the antimicrobial rate is calculated with the following formula, in which the value has two significant digits after rounding up or down the third significant digit:

$$R(\%)=(B-S)/B \times 100$$

wherein

R: antimicrobial rate (%)
B: mean recovery viable counts of crude test pieces (cfu/piece)
S: mean recovery viable counts of antimicrobial product test pieces (cfu/piece)

**SPECIFIC EMBODIMENTS**

In an aspect, the present application is directed to an antimicrobial coating comprising an antimicrobial effective amount of silver powders and stainless steel powders, wherein the silver powders and the stainless steel powders are present in the form of physical doping in the antimicrobial coating.

In some preferred embodiments, the antimicrobial coating comprises an antimicrobial effective amount of silver powders, copper powders and stainless steel powder, wherein the silver powders, the copper powders and the stainless steel powders are present in the form of physical doping in the antimicrobial coating.

In some embodiments, the antimicrobial coating comprises about 0.01-30% silver powders and stainless steel powders, wherein the silver powders and the stainless steel powders are present in the form of physical doping in the antimicrobial coating.

In some preferred embodiments, the antimicrobial coating comprises an antimicrobial effective amount of silver powders and stainless steel powders, wherein the silver powders and the stainless steel powders are present in the form of physical doping in the antimicrobial coating and the ratio of the silver powders to the stainless steel powders is about 1-20% by weight.

In some preferred embodiments, the antimicrobial coating comprises an antimicrobial effective amount of silver powders and stainless steel powders, wherein the silver powders and the stainless steel powders are present in the form of physical doping in the antimicrobial coating and the ratio of the silver powder to the stainless steel powder is about 5-10% by weight.

In some embodiments, the antimicrobial coating comprises an antimicrobial effective amount of silver powders, copper powders and stainless steel powders, wherein the silver powders, the copper powders and the stainless steel powders are present in the form of physical doping in the antimicrobial coating and the ratio of the copper powder to the stainless steel powder is about 0.1-40% by weight.

In some embodiments, the antimicrobial coating comprises an antimicrobial effective amount of silver powders, copper powders and stainless steel powders, wherein the silver powders, the copper powders and the stainless steel powders are present in the form of physical doping in the antimicrobial coating, the ratio of the silver powders to the stainless steel powders is about 0.01-30% by weight and the ratio of the copper powder to stainless steel powder is about 0.1-40% by weight.

In some embodiments, the antimicrobial coating comprises an antimicrobial effective amount of silver powders and nickel-based containing chromium stainless steel powders, wherein the silver powders and the nickel-based containing chromium stainless steel powders are present in the form of physical doping in the antimicrobial coating.

In some embodiments, the antimicrobial coating comprises an antimicrobial effective amount of silver powders and cobalt-based containing chromium stainless steel powders, wherein the silver powders and the cobalt-based containing chromium stainless steel powders are present in the form of physical doping in the antimicrobial coating.

In some embodiments, the antimicrobial coating comprises an antimicrobial effective amount of silver powders and the iron-based containing chromium stainless steel powders, wherein the silver powders and the iron-base containing chromium stainless steel powders are present in the form of physical doping in the antimicrobial coating.

In some embodiments, the antimicrobial coating comprises an antimicrobial effective amount of silver powders with the particle size of about 200 to 2000 mesh and stainless steel powders, wherein the silver powders and the stainless steel powders are present in the form of physical doping in the antimicrobial coating.

In some preferred embodiments, the antimicrobial coating comprises about 0.01-30% silver powders with the particle size of about 200 to 2000 mesh and stainless powders, wherein the silver powders and the stainless steel powders are present in the form of physical doping in the antimicrobial coating, and the stainless steel powders are nickel-based containing chromium stainless steel powders, cobalt-based containing chromium stainless steel powders, iron-based containing chromium stainless steel powders or any mixture thereof.

In some more preferred embodiments, the antimicrobial coating comprises about 1-20% silver powders with the particle size of about 200 to 2000 mesh and stainless powders, wherein the silver powders and the stainless steel powders are present in the form of physical doping in the antimicrobial coating, and the stainless steel powders are nickel-based containing chromium stainless steel powders, cobalt-based containing chromium stainless steel powders, iron-based containing chromium stainless steel powders or any mixture thereof.

In some more preferred embodiments, the antimicrobial coating comprises about 5-10% silver powders with the particle size of about 200 to 2000 mesh and stainless powders, wherein the silver powders and the stainless steel powders are present in the form of physical doping in the antimicrobial coating, and the stainless steel powders are nickel-based containing chromium stainless steel powders, cobalt-based containing chromium stainless steel powders, iron-based containing chromium stainless steel powders or any mixture thereof.

In some even more preferred embodiments, the antimicrobial coating comprises about 0.01-30% silver powders with the particle size of about 200 to 2000 mesh, about 0.1-40% copper powders with the particle size of about 200 to 2000 mesh and stainless steel powders, wherein the silver powders, the copper powders and the stainless steel powders are present in the form of physical doping in the antimicrobial coating, and the stainless steel powders are nickel-based containing chromium stainless steel powders, cobalt-based containing chromium stainless steel powders, iron-based containing chromium stainless steel powders or any mixture thereof.

In some embodiments, the antimicrobial coating further comprises trace amount of elements beneficial to the human body.

The examples of elements beneficial to the human body which can be contained in the antimicrobial coating of the present application include, but are not limited to, potassium, calcium, zinc, chromium, nickel, cobalt, manganese, iron, magnesium, molybdenum, titanium and any mixture thereof.

In some embodiments, the antimicrobial coating comprises an antimicrobial effective amount of silver powders and stainless steel powders, wherein the silver powders and the stainless steel powders are present in the form of physical doping in the antimicrobial coating and the antimicrobial coating further comprises trace amount of powdery potassium, calcium, zinc, chromium, nickel, cobalt, manganese, iron, magnesium, molybdenum, titanium and any mixture thereof.

In some preferred embodiments, the antimicrobial coating comprises about 0.01-30% silver powders and stainless steel powders, wherein the silver powders and the stainless steel powders are present in the form of physical doping in the antimicrobial coating and the antimicrobial coating further comprises trace amount of powdery potassium, calcium, zinc, chromium, nickel, cobalt, manganese, iron, magnesium, molybdenum, titanium and any mixture thereof.

In some preferred embodiments, the antimicrobial coating comprises an antimicrobial effective amount of silver powders with the particle size of about 200 to 2000 mesh and stainless steel powders, wherein the silver powders and the stainless steel powders are present in the form of physical doping in the antimicrobial coating and the antimicrobial coating further comprises trace amount of powdery potassium, calcium, zinc, chromium, nickel, cobalt, manganese, iron, magnesium, molybdenum, titanium and any mixture thereof.

In some more preferred embodiments, the antimicrobial coating comprises about 0.01-30% silver powders with the particle size of about 200 to 2000 mesh, about 0.1-40% copper powders with the particle size of about 200 to 2000 mesh and stainless steel powders, wherein the silver powders, the copper powders and the stainless steel powders are present in the form of physical doping in the antimicrobial coating and the antimicrobial coating further comprises trace amount of powdery potassium, calcium, zinc, chromium, nickel, cobalt, manganese, iron, magnesium, molybdenum, titanium and any mixture thereof.

In some more preferred embodiments, the antimicrobial coating comprises about 0.01-30% silver powders with the particle size of about 200 to 2000 mesh and stainless steel powders, wherein the silver powders and the stainless steel powders are present in the form of physical doping in the antimicrobial coating, the stainless steel powders are nickel-based containing chromium stainless steel powders, cobalt-based containing chromium stainless steel powders, iron-based containing chromium stainless steel powders or any mixture thereof and the antimicrobial coating further comprises trace amount of powdery potassium, calcium, zinc, chromium, nickel, cobalt, manganese, iron, magnesium, molybdenum, titanium and any mixture thereof.

In some more preferred embodiments, the antimicrobial coating comprises about 0.01-30% silver powders with the particle size of 200 to 2000 mesh, about 0.1-40% copper powders with the particle size of 200 to 2000 mesh and stainless steel powders, wherein the silver powders, the copper powders and the stainless steel powders are present in the form of physical doping in the antimicrobial coating, the stainless steel powders are nickel-based containing chromium stainless steel powders, cobalt-based containing chromium stainless steel powders, iron-based containing chromium stainless steel powders or any mixture thereof and the antimicrobial coating further comprises trace amount of powdery potassium, calcium, zinc, chromium, nickel, cobalt, manganese, iron, magnesium, molybdenum, titanium and any mixture thereof.

In another aspect, the present application is directed to a metal product, wherein the surface of a substrate is coated with an antimicrobial coating comprising an antimicrobial effective amount of silver powders and stainless steel powders, wherein the silver powders and stainless steel powders are present in the form of physical doping in the antimicrobial coating.

According to the functions and properties of the metal product, any suitable substrates can be coated with the antimicrobial coating of the present application.

The examples of the substrates which can be used in the present application include, but are not limited to, a ferrous metal, a non-ferrous metal, an alloy and a stainless steel.

The examples of the ferrous metal which can be used as the substrate of the present application include, but are not limited to, iron, manganese and chromium.

The examples of the non-ferrous metal which can be used as the substrate of the present application include, but are not limited to, copper, nickel, cobalt, lead, zinc, tin, antimony, titanium, zirconium, molybdenum, tungsten, scandium and yttrium.

The examples of the alloy which can be used as the substrate of the present application include, but are not limited to, steel, aluminum alloy, copper alloy, magnesium alloy, nickel alloy, tin alloy, tantalum alloy, titanium alloy, zinc alloy, molybdenum alloy and zirconium alloy.

The examples of the stainless steel which can be used as the substrate of the present application include, but are not limited to, a martensite type stainless steel, an austenite type stainless steel, a ferrite type stainless steel, a duplex type stainless steel, and the like.

The examples of the metal product related to the present application include, but are not limited to, a kitchen utensil, a medical appliance, and the like.

In some embodiments, the examples of the kitchen utensil include, but are not limited to, cutter, cooking utensil, slice, grilling tools, chopsticks, and the like.

In other embodiments, the examples of the medical appliance include, but are not limited to, medical device, scalpel, hemostat, finger pliers, orthopedic tweezers, anatomic tweezers, dressing tweezers, anatomic tweezers, dissecting needle, nervous percussion hammer, ophthalmic surgical scissors, eyelash tweezers, ophthalmic tweezers, throat sprayer, throat mirror, tuning fork, dental tweezers, dental probe, trumpet anoscope, and the like.

In some preferred embodiments, the antimicrobial coating coated on the surface of the substrate of a metal product comprises an antimicrobial effective amount of silver powders, copper powders and stainless steel powders, wherein the silver powders, the copper powders and the stainless steel powders are present in the form of physical doping in the coating.

In some embodiments, the antimicrobial coating coated on the surface of the substrate of a metal product comprises about 0.01-30% silver powders and stainless steel powders, wherein the silver powders and the stainless steel powders are present in the form of physical doping in the coating.

In some preferred embodiments, the antimicrobial coating coated on the surface of the substrate of a metal product comprises about 1-20% silver powders and stainless steel powders, wherein the silver powders and the stainless steel powders are present in the form of physical doping in the coating.

In some preferred embodiments, the antimicrobial coating coated on the surface of the substrate of a metal product comprises about 5-10% silver powders and stainless steel powders, wherein the silver powders and the stainless steel powders are present in the form of physical doping in the coating.

In some preferred embodiments, the antimicrobial coating coated on the surface of the substrate of a metal product comprises about 0.01-30% silver powders, about 0.1-40% copper powders and stainless steel powders, wherein the silver powders, the copper powders and the stainless steel powders are present in the form of physical doping in the coating.

In some embodiments, the antimicrobial coating coated on the surface of the substrate of a metal product comprises an antimicrobial effective amount of silver powders with the particle size of about 200 to 2000 mesh and stainless steel powders, wherein the silver powders and the stainless steel powders are present in the form of physical doping in the coating.

In some preferred embodiments, the antimicrobial coating coated on the surface of the substrate of a metal product comprises about 0.01-30% silver powders with the particle size of about 200 to 2000 mesh and stainless steel powders, wherein the silver powders and the stainless steel powders are present in the form of physical doping in the coating.

In some more preferred embodiments, the antimicrobial coating coated on the surface of the substrate of a metal product comprises about 0.01-30% silver powders with the particle size of about 200 to 2000 mesh, about 0.1-40% copper powders with the particle size of about 200 to 2000 mesh and stainless steel powders, wherein the silver powders, the copper powders and the stainless steel powders are present in the form of physical doping in the coating.

In some embodiments, the substrate coated with an antimicrobial coating of the present application can be a martensite type stainless steel. The examples of the martensite type stainless steel include, but art not limited to, 403, 410, 414, 416, 416Se, 420, 431, 440A, 440B and 440C type stainless steels.

In some embodiments, the substrate coated with an antimicrobial coating of the present application can be an austenite type stainless steel. The examples of the austenite type stainless steel include, but art not limited to, 201, 202, 205, 301, 302, 302B, 303, 303Se, 304, 304L, 302HQ, 304N, 305, 308 and 309 type stainless steels.

In some embodiments, the substrate coated with an antimicrobial coating of the present application can be a ferrite type stainless steel. The examples of the ferrite type stainless steel include, but art not limited to, 405, 409, 429, 430 and 430F type stainless steels.

In some embodiments, the substrate coated with an antimicrobial coating of the present application can be a duplex type stainless steel. The examples of the duplex type stainless steel include, but art not limited to, 329 and 2205 type stainless steels.

In some more preferred embodiments, the antimicrobial coating coated on the surface of the substrate of a metal product comprises about 0.01-30% silver powders with the particle size of about 200 to 2000 mesh, copper powders and stainless powders, wherein the silver powders, the copper powders and the stainless steel powders are present in the form of physical doping in the antimicrobial coating, the stainless steel powders are nickel-based containing chromium stainless steel powders, cobalt-based containing chromium stainless steel powders, iron-based containing chromium stainless steel powders or any mixture thereof.

In some even more preferred embodiments, the antimicrobial coating coated on the surface of the substrate of a metal product comprises about 0.01-30% silver powders with the particle size of about 200 to 2000 mesh, about 0.1-40% copper powders with the particle size of about 200 to 2000 mesh and stainless steel powders, wherein the silver powders, the copper powders and the stainless steel powders are present in the form of physical doping in the antimicrobial coating, and the stainless steel powders are nickel-based containing chromium stainless steel powders, cobalt-based containing chromium stainless steel powders, iron-based containing chromium stainless steel powders or any mixture thereof.

In some embodiments, the antimicrobial coating coated on the surface of the substrate of a metal product further comprises trace amount of elements beneficial to the human body.

The examples of elements beneficial to the human body which can be contained in the antimicrobial coating coated on the surface of the substrate of a metal product of the present application include, but are not limited to, potassium, calcium, zinc, chromium, nickel, cobalt, manganese, iron, magnesium, molybdenum, titanium and any mixture thereof.

In some embodiments, the antimicrobial coating coated on the surface of the substrate of a metal product comprises an antimicrobial effective amount of silver powders and stainless steel powders, wherein the silver powders and the stainless steel powders are present in the form of physical doping in the antimicrobial coating and the antimicrobial coating further comprises trace amount of powdery potassium, calcium, zinc, chromium, nickel, cobalt, manganese, iron, magnesium, molybdenum, titanium or any mixture thereof.

In some preferred embodiments, the antimicrobial coating coated on the surface of the substrate of a metal product comprises about 0.01-30% of silver powders and stainless steel powders, wherein the silver powders and the stainless steel powders are present in the form of physical doping in the antimicrobial coating and the antimicrobial coating further comprises trace amount of powdery potassium, calcium, zinc, chromium, nickel, cobalt, manganese, iron, magnesium, molybdenum, titanium or any mixture thereof.

In some preferred embodiments, the antimicrobial coating coated on the surface of the substrate of a metal product comprises an antimicrobial effective amount of silver powders with the particle size of about 200 to 2000 mesh and the stainless steel powders, wherein the silver powders and the stainless steel powders are present in the form of physical doping in the antimicrobial coating and the antimicrobial coating further comprises trace amount of powdery potassium, calcium, zinc, chromium, nickel, cobalt, manganese, iron, magnesium, molybdenum, titanium or any mixture thereof.

In some more preferred embodiments, the antimicrobial coating coated on the surface of the substrate of a metal product comprises 0.01-30% silver powders with the particle size of about 200 to 2000 mesh and stainless steel powders, wherein the silver powders and the stainless steel powders are present in the form of physical doping in the antimicrobial coating and the antimicrobial coating further comprises trace amount of powdery potassium, calcium, zinc, chromium, nickel, cobalt, manganese, iron, magnesium, molybdenum, titanium or any mixture thereof.

In some more preferred embodiments, the antimicrobial coating coated on the surface of the substrate of a metal product comprises 0.01-30% silver powders with the particle size of about 200 to 2000 mesh, about 0.1-40% copper powders with the particle size of about 200 to 2000 mesh and stainless steel powders, wherein the silver powders, the copper powders and the stainless steel powders are present in the form physical doping in the antimicrobial coating, the stainless steel powders are nickel-based containing chromium stainless steel powders, cobalt-based containing chromium stainless steel powders, iron-based containing chromium stainless steel powders or any mixture thereof, and the antimicrobial coating further comprises trace amount of powdery potassium, calcium, zinc, chromium, nickel, cobalt, manganese, iron, magnesium, molybdenum, titanium or any mixture thereof.

In still another aspect, the present application is directed to a process for preparing an antimicrobial coating, comprising:

(1) mixing an antimicrobial effective amount of silver powders and stainless steel powders; and
(2) spraying the mixture of the silver powders and the stainless steel powders on a surface to be processed,

wherein the silver powders and the stainless steel powders are present in the form of physical doping in the antimicrobial coating.

In some embodiments, the antimicrobial effective amount of silver powder is mixed with nickel-base containing chromium stainless steel powder, cobalt-base containing chromium stainless steel powder, iron-base containing chromium stainless steel powder or any mixture thereof, and melt sprayed on the surface to be processed so as to prepare the antimicrobial coating.

In some embodiments, the process for preparing the antimicrobial coating further comprises sandblasting the surface to be processed before spraying.

In some embodiments, the Ra of the surface is about 0.7-5 μm after sandblasting.

In some preferred embodiments, the Ra of the surface is about 1.5-2.5 μm after sandblasting.

In some embodiments, the process for preparing the antimicrobial coating further comprises polishing the obtained antimicrobial coating.

In some preferred embodiments, a process for preparing an antimicrobial coating comprises:

(1) mixing an antimicrobial effective amount of silver powders with nickel-based containing chromium stainless steel powders, cobalt-based containing chromium stainless steel powders, iron-based containing chromium stainless steel powders or any mixtures thereof;
(2) sandblasting a surface to be processed;
(3) spraying the mixture of the silver powders and the nickel-based containing chromium stainless steel powders, cobalt-based containing chromium stainless steel powders, iron-based containing chromium stainless steel powders or any mixture thereof on the surface to be processed; and
(4) polishing the obtained antimicrobial coating,

wherein the silver powders and the stainless steel powders are present in the form of physical doping in the antimicrobial coating.

In some embodiments, the thickness of the polished coating is about 0.001-0.35 mm.

In some embodiments, the spraying is carried out with high velocity oxy-fuel spraying or high-speed low-temperature spraying.

In some preferred embodiments, the velocity of the particles during spraying is no less than about 100 m/s.

In some embodiments, a process for preparing an antimicrobial coating comprises:

(1) mixing an antimicrobial effective amount of silver powders, copper powders and stainless steel powders; and
(2) spraying the mixture of the silver powders, the copper powders and the stainless steel powders on a surface to be processed,

wherein the silver powders, the copper powders and the stainless steel powders are present in the form of physical doping in the antimicrobial coating.

In some embodiments, an antimicrobial effective amount of silver powders, copper powders and nickel-based containing chromium stainless steel powders, cobalt-based containing chromium stainless steel powders, iron-based containing chromium stainless steel powders or any mixture thereof are mixed, and then the resultant mixture is sprayed on a surface to be processed so as to prepare an antimicrobial coating.

In some preferred embodiments, a process for preparing an antimicrobial coating comprises:

(1) about 0.01-30% silver powder and about 0.1-40% copper powder are mixed with the stainless steel powder; and
(2) the mixture of the silver powder, copper powder and stainless steel powder is melt sprayed on the surface to be processed,

wherein the silver powder, copper powder and stainless steel powder are present in the antimicrobial coating in the form of physical doping.

In some embodiments, about 0.01-30% silver powders, about 0.1-40% copper powders are mixed with nickel-based containing chromium stainless steel powders, cobalt-based containing chromium stainless steel powders, iron-based containing chromium stainless steel powders or any mixtures thereof, and then the resultant mixture is sprayed on a surface to be processed so as to prepare an antimicrobial coating.

In some preferred embodiments, a process for preparing an antimicrobial coating comprises:

(1) mixing about 0.01-30% silver powders with stainless steel powders and adding trace amount of powder elements beneficial to the human body; and
(2) spraying the mixture of the silver powders, the stainless steel powders and the powdery elements beneficial to the human body on a surface to be processed,

wherein the silver powders, the stainless steel powders and the powdery elements beneficial to the human body are present in the form of physical doping in the antimicrobial coating.

In some embodiments, an antimicrobial effective amount of silver powders, trace amount of powdery elements beneficial to the human body are mixed with nickel-based containing chromium stainless steel powders, cobalt-based containing chromium stainless steel powders, iron-based containing chromium stainless steel powders or any mixture thereof, and then the resultant mixture is sprayed on a surface to be processed so as to prepare an antimicrobial coating.

In some more preferred embodiments, a process for preparing an antimicrobial coating comprises:

(1) mixing about 0.01-30% silver powders with the particle size of about 200 to 2000 mesh with stainless steel powders and adding trace amount of powdery elements beneficial to the human body;
(2) sandblasting a surface to be processed;
(3) spraying the mixture of the silver powders, the stainless steel powders and the powdery elements beneficial to the human body on the surface to be processed with high velocity oxy-fuel spraying or high-speed low-temperature spraying; and
(4) polishing the obtained antimicrobial coating,

wherein the silver powders, the stainless steel powders and the powdery elements beneficial to the human body are present in the form of physical doping in the antimicrobial coating.

In some embodiments, the antimicrobial metal, the stainless steel and the added powdery elements beneficial to the body are powdery or in the form of flux-cored wire prepared with powders before spraying.

In yet another aspect, the present application is directed to an antimicrobial coating prepared with the following process comprising:

(1) mixing an antimicrobial effective amount of silver powders and stainless steel powders; and
(2) spraying the mixture of the silver powders and the stainless steel powders on a surface to be processed,

wherein the silver powders and the stainless steel powders are present in the form of physical doping in the antimicrobial coating.

In some embodiments, the antimicrobial effective amount of silver powder is mixed with nickel-base containing chromium stainless steel powder, cobalt-base containing chromium stainless steel powder, iron-base containing chromium stainless steel powder or any mixture thereof, and then melt sprayed on the surface to be processed so as to prepare the antimicrobial coating, wherein the silver powder and stainless steel powder are present in the antimicrobial coating in the form of physical doping

In some embodiments, the process for preparing an antimicrobial coating further comprises sandblasting the surface to be processed before spraying.

In some embodiments, the Ra of the surface is about 0.7-5 $\mu$m after sandblasting.

In some preferred embodiments, the Ra of the surface is about 1.5-2.5 $\mu$m after sandblasting.

In some embodiments, the process for preparing an antimicrobial coating further comprises polishing the obtained antimicrobial coating.

In some preferred embodiments, an antimicrobial coating is prepared with the following process comprising:

(1) mixing an antimicrobial effective amount of silver powders with nickel-based containing chromium stainless steel powders, cobalt-based containing chromium stainless steel powders, iron-based containing chromium stainless steel powders or any mixture thereof;
(2) sandblasting a surface to be processed;
(3) spraying the mixture of the silver powders and the nickel-based containing chromium stainless steel powders, cobalt-based containing chromium stainless steel powders, iron-based containing chromium stainless steel powders or any mixtures thereof on the surface to be processed; and
(4) polishing the obtained antimicrobial coating,

wherein the silver powders and the stainless steel powders are present in the form of physical doping in the antimicrobial coating.

In some embodiments, the thickness of the polished coating is about 0.001-0.35 mm.

In some embodiments, the spraying is carried out with high velocity oxy-fuel spraying or high-speed low-temperature spraying.

In some preferred embodiments, the velocity of the particles during spraying is no less than about 100 m/s.

In some embodiments, an antimicrobial coating is prepared with the following process comprising:

(1) mixing an antimicrobial effective amount of silver powders, copper powders and stainless steel powders; and
(2) spraying the mixture of the silver powders, the copper powders and the stainless steel powders on a surface to be processed,

wherein the silver powders, the copper powders and the stainless steel powders are present in the form of physical doping in the antimicrobial coating.

In some embodiments, an antimicrobial effective amount of silver powders, copper powders are mixed with nickel-based containing chromium stainless steel powders, cobalt-based containing chromium stainless steel powders, iron-based containing chromium stainless steel powders or any mixture thereof, and then the resultant mixture is sprayed on a surface to be processed so as to prepare an antimicrobial coating, wherein the silver powders, the copper powders and the stainless steel powders are present in the form of physical doping in the antimicrobial coating.

In some preferred embodiments, an antimicrobial coating is prepared with the following process comprising:

(1) mixing about 0.01-30% silver powders and about 0.1-40% copper powders with the stainless steel powders; and
(2) spraying the mixture of the silver powders, the copper powders and the stainless steel powders on a surface to be processed,

wherein the silver powders, the copper powders and the stainless steel powders are present in the form of physical doping in the antimicrobial coating.

In some embodiments, about 0.01-30% silver powders, about 0.1-40% copper powders are mixed with nickel-based containing chromium stainless steel powders, cobalt-based containing chromium stainless steel powders, iron-based containing chromium stainless steel powders or any mixture thereof, and then the resultant mixture is sprayed on a surface to be processed so as to prepare an antimicrobial coating, wherein the silver powders, the copper powders and the stainless steel powders are present in the form of physical doping in the antimicrobial coating.

In some preferred embodiments, an antimicrobial coating is prepared with the following process comprising:

(1) mixing about 0.01-30% silver powders and stainless steel powders and then adding trace amount of elements beneficial to the human body; and
(2) spraying the mixture of the silver powders, the stainless steel powders and the powdery elements beneficial to the human body on a surface to be processed,

wherein the silver powder stainless steel powders and the powdery elements beneficial to the human body are present coating in the form of physical doping in the antimicrobial.

In some embodiments, an antimicrobial effective amount of the silver powders, powdery elements beneficial to the body are mixed with nickel-based containing chromium stainless steel powders, cobalt-based containing chromium stainless steel powders, iron-based containing chromium stainless steel powders or any mixture thereof, and then the resultant mixture is sprayed on a surface to be processed so as to prepare an antimicrobial coating, wherein the silver powder stainless steel powders and the powdery elements beneficial to the human body are present in the form of physical doping in the antimicrobial coating.

In some more preferred embodiments, an antimicrobial coating is prepared with the following process comprising:

(1) mixing about 0.01-30% silver powders with the particle size of about 200 to 2000 mesh with stainless steel powders and adding trace amount of powdery elements beneficial to the human body;
(2) sandblasting a surface to be processed;
(3) spraying the mixture of the silver powders, the stainless steel powders and the powdery elements beneficial to the human body on the surface to be processed with high velocity oxy-fuel spraying or high-speed low-temperature spraying; and
(4) polishing the obtained antimicrobial coating,

wherein the silver powders, the stainless steel powders and the powdery elements beneficial to the human body are present in the form of physical doping in the antimicrobial coating.

In some more preferred embodiments, an antimicrobial coating is prepared with the following process comprising:

(1) mixing about 0.01-30% silver powders with the particle size of about 200 to 2000 mesh, about 0.1-40% copper powders with the particle size of about 200 to 2000 mesh are mixed with stainless steel powders and adding trace amount of powdery elements beneficial to the human body;
(2) sandblasting a surface to be processed;
(3) spraying the mixture of the silver powders, the copper powders, the stainless steel powders and the powdery elements beneficial to the human body on the surface to be processed with high velocity oxy-fuel spraying or high-speed low-temperature spraying; and
(4) polishing the obtained antimicrobial coating,

wherein the silver powders, the copper powders, the stainless steel powders and the powdery elements beneficial to the human body are present in the form of physical doping in the antimicrobial coating.

In still another aspect, the present application is directed to a process for protecting against microbes comprising applying an antimicrobial coating which comprises an antimicrobial effective amount of silver powders and stainless steel powders to a product in need of antimicrobial action, wherein the silver powders and the stainless steel powders are present in the form of physical doping in the antimicrobial coating.

Hereafter, detailed illustration will be carried out through the following examples referring to accompanying figures for better understanding of the various aspects and advantages of the present invention. However, it will be appreciated that the following examples are non-limiting and only for illustrating some embodiments of the present invention.

## EXAMPLES

### Example 1

0.5 g metal silver powders were mixed with 99.5 g nickel-based containing chromium stainless steel powders (NiCr-Cr$_3$C$_2$) in a suitable container. The surface to be processed was sandblasted and cleaned so that the surface roughness Ra reached 0.7-5 $\mu$m. With the high velocity oxy-fuel spraying (HVAF or HVOF), the obtained antimicrobial metal coating was polished at a high speed (the velocity of the particles was 160 m/s during spraying). The thickness of the polished coating was 0.30 mm.

### Example 2

0.1 g metal silver powders were mixed with 99.0 g nickel-based containing chromium stainless steel powders (NiCr-Cr$_3$C$_2$) in a suitable container. The surface to be processed was sandblasted and cleaned so that the surface roughness Ra reached 0.7-5 $\mu$m. With the high-speed low-temperature spraying, the obtained antimicrobial metal coating was polished at a high speed (the velocity of particle was 110 m/s during spraying). The thickness of the polished coating was 0.20 mm.

### Example 3

5.0 g metal silver powders were mixed with 95.0 g cobalt-based containing chromium stainless steel powders in a suitable container. The surface to be processed was sandblasted and cleaned so that the surface roughness Ra reached 0.7-5 $\mu$m. With the high-speed low-temperature spraying, the obtained antimicrobial metal coating was polished at a high speed (the velocity of particle was 150 m/s during spraying). The thickness of the polished coating was 0.10 mm.

### Example 4

The antimicrobial effects of the obtained antimicrobial coatings in examples 2 and 3, Japanese antimicrobial stainless steels (standard No.: JIS Z 2801 (2000)) and conventional stainless steels were tested with the antimicrobial performance test method described in the present application. The results are listed in Table 2.

**Table 2**

| strains / categories | *Escherichia coli* | *Staphylococcus aureus* | *Pseudomonas aeruginosa* | *Salmonella aertrycke* | *Klebsiella pneumoniae* |
|---|---|---|---|---|---|
| Japanese antimicrobial stainless steels standard No.: | ≥ 99.00% | ≥ 99.00% | ≥ 99.00% | ≥ 99.00% | ≥ 99.00% |

| JIS Z 2801 (2000) | | | | | |
|---|---|---|---|---|---|
| conventional stainless steels | < 90.00% | < 90.00% | < 90.00% | < 90.00% | < 90.00% |
| Example 2 stainless steel + 1% silver | ≥ 99.97% | ≥ 99.98% | - | - | ≥ 99.98% |
| Example 2 stainless steel + 5% silver | ≥ 99.98% | ≥ 99.98% | ≥ 99.92% | ≥ 99.98% | ≥ 99.98% |

In view of the results as listed in Table 2, the antimicrobial coatings containing 1% silvers of the present application can achieve the antimicrobial effects of more than 99.9%. Therefore, the antimicrobial coatings of the present application have better antimicrobial effects than thaose of Japanese antimicrobial stainless steels and the conventional stainless steels.

Moreover, since the antimicrobial coating of the present application is in the form of physical doping, the antimicrobial metal powders are independently present in the metal coatings. The antimicrobial coatings of the present application have excellent properties such as corrosion resistance, high-temperature resistance, and the like on the basis of the properties of the antimicrobial metals (for example, silver has a melting point of 961.93 °C, and excellent flexibility and ductility. Silver is also not reactive with water or oxygen in the air under ambient temperature or even being heated).

These and other changes can be made in light of the above-detailed description. In general, in the following claims, the terms used should be not construed to be limiting to the specific embodiments disclosed in the specification and claims, but should be construed to include all systems, devices and/or methods that operate in accordance with the claims. Accordingly, the scope of the present invention is not limited by the disclosure, but instead its scope is to be determined entirely by the following claims.

All of the above US patents, US patent application publications, US patent applications, foreign patents, foreign patent applications and non-patent publications referred to in this specification are incorporated herein by reference in their entirety.

From the forgoing it will be appreciated, although specific embodiments of the present invention have been described herein for purposes of illustration, various modifications may be made without deviating from the spirit and scope of the present invention. Accordingly, the present invention is only limited except as by the appended claims.

**Claims**

1. An antimicrobial coating, comprising an antimicrobial effective amount of silver powders and stainless steel powders, wherein the silver powders and the stainless steel powders are present in the form of physical doping in the antimicrobial coating.

2. The antimicrobial coating of claim 1, wherein the antimicrobial coating further comprises copper powders.

3. The antimicrobial coating of claim 1 or 2, wherein the stainless steel powders are nickel-based containing chromium stainless steel powders, cobalt-based containing chromium stainless steel powders, iron-based containing chromium stainless steel powders or any mixture thereof.

4. The antimicrobial coating of any one of claims 1 to 3, wherein the ratio of the silver powders to the stainless steel powders is about 0.01-30% by weight.

5. The antimicrobial coating of any one of claims 2 to 4, wherein the ratio of the copper powders to the stainless steel powders is about 0.1-40% by weight.

6. The antimicrobial coating of any one of claims 1 to 5, wherein the particle size of the silver powders is about 200 to 2000 mesh.

7. The antimicrobial coating of any one of claims 1 to 6, wherein the antimicrobial coating further comprises trace amount of powdery elements beneficial to the human body.

8. The antimicrobial coating of claim 7, wherein the powdery elements beneficial to the human body are selected from the group consisting of potassium, calcium, zinc, chromium, nickel, cobalt, manganese, iron, magnesium, molybdenum, titanium and any mixture thereof.

9. A metal product, wherein the surface of a substrate is coated with the coating of any one of claims 1 to 8.

10. The metal product of claim 9, wherein the substrate is selected from the group consisting of a ferrous metal, a non-ferrous metal, an alloy and a stainless steel.

11. The metal product of claim 9 or 10, wherein the metal product is a kitchen utensil, and preferably a cutter, a cooking utensil, a slice, grilling tools or chopsticks.

12. The metal product of claim 9 or 10, wherein the metalwork is medical appliance, preferably a medical device, a scalpel, a hemostat, finger pliers, orthopedic tweezers, anatomic tweezers, dressing tweezers, anatomic tweezers, a dissecting needle, a nervous percussion hammer, ophthalmic surgical scissors, eyelash tweezers, ophthalmic tweezers, a throat sprayer, a throat mirror, a tuning fork, dental tweezers, a dental probe or a trumpet anoscope.

13. The metal product of any one of claims 9 to 12, wherein the thickness of the antimicrobial coating is about 0.001-0.35 mm.

14. A process for preparing the antimicrobial coating of any one of claims 1 to 8, comprising:

    (1) mixing an antimicrobial amount of silver powders and stainless steel powders; and
    (2) spraying the mixture of the silver powders and the stainless steel powders on a surface to be processed.

15. The process of claim 14, further comprising sandblasting the surface to be processed before spraying.

16. The process of claim 15, wherein the Ra of the surface is about 0.7-5 $\mu$m after sandblasting.

17. The process of claim 14, further comprising polishing the obtained antimicrobial coating.

18. The process of claim 17, wherein the thickness of the polished coating is about 0.001-0.35 mm.

19. The process of any one of claims 14 to 18, wherein spraying is carried out with high velocity oxy-fuel spraying or high-speed low-temperature spraying.

20. The process of any one of claims 14 to 19, wherein the velocity of particles during spraying is no less than about 100 m/s.

21. The process of any one of claims 14 to 20, wherein the antimicrobial metal, stainless steel and the added elements beneficial to the human body are powdery or in the form of a flux-cored wire prepared by powders before spraying.

22. An antimicrobial coating prepared with the following process comprising:

(1) mixing an antimicrobial effective amount of silver powders and stainless steel powders; and
(2) spraying the mixture of the silver powders and the stainless steel powders on a surface to be processed,

wherein the silver powders and the stainless steel powders are present in the form of physical doping in the antimicrobial coating.

23. A process for protecting against microbes, comprising applying the coating of any one of claims 101 to 108 to a product in need of antimicrobial action.

antibacterial coatings

2000 mesh

200 mesh

substrate

substrate

# Fig 1

Fig 2

Fig 3

# INTERNATIONAL SEARCH REPORT

| | International application No. |
|---|---|
| | PCT/CN2008/073544 |

**A. CLASSIFICATION OF SUBJECT MATTER**

See extra sheet

According to International Patent Classification (IPC) or to both national classification and IPC

**B. FIELDS SEARCHED**

Minimum documentation searched (classification system followed by classification symbols)

IPC: C23C4/-; C03C17/-; C04B41/-; C23C14/-

Documentation searched other than minimum documentation to the extent that such documents are included in the fields searched

Electronic data base consulted during the international search (name of data base and, where practicable, search terms used)

WPI; EPODOC; PAJ; CNKI; CNPAT; stainless steel, silver, copper, melt spray+, spray+, coat+, antimicrob+, antibacterial, antibio+, antisep+, Ag, Cu

**C. DOCUMENTS CONSIDERED TO BE RELEVANT**

| Category* | Citation of document, with indication, where appropriate, of the relevant passages | Relevant to claim No. |
|---|---|---|
| X | JP59067364A (TSUKAMOTO SEIKI KK) 17 Apr. 1984 (17.04.1984) Description, page 2 | 1, 3-6, 9-23 |
| Y | | 2, 7, 8 |
| Y | CN1246455A (JOINT LOGISTICS DEPARTMENT OF PLA SHENYANG MILITARY REGION, SANITATION AND ANTIEPIDEMIC TEAMS) 08 Mar. 2000 (08.03.2000) Description, page 1 lines 21-31 | 2, 7, 8 |
| X | GB2366613A (TSAO Mindy) 13 Mar. 2002 (13.03.2002) Claims 2 and 3 | 1-13, 23 |
| A | EP0140032A1 (PPG INDUSTRIES INC.) 08 May 1985 (08.05.1985) Claims 1-16 | 1-23 |
| A | JP2005002408A (HITACHI LTD.) 06 Jan. 2005 (06.01.2005) Paragraph [0014] | 1-23 |
| A | WO2004094685A2 (DIAMOND INNOVATIONS INC.) 04 Nov. 2004 (04.11.2004) Claims 1-7 | 1-23 |

☐ Further documents are listed in the continuation of Box C.  ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim (S) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 30 Aug. 2009 (30.08.2009) | **24 Sep. 2009 (24.09.2009)** |

| Name and mailing address of the ISA/CN The State Intellectual Property Office, the P.R.China 6 Xitucheng Rd., Jimen Bridge, Haidian District, Beijing, China 100088 Facsimile No. 86-10-62019451 | Authorized officer DAI Lingli Telephone No. (86-10)62413986 |
|---|---|

Form PCT/ISA/210 (second sheet) (April 2007)

## INTERNATIONAL SEARCH REPORT
Information on patent family members

International application No.

PCT/CN2008/073544

| Patent Documents referred in the Report | Publication Date | Patent Family | Publication Date |
|---|---|---|---|
| JP59067364A | 17.04.1984 | JP59034787B | 24.08.1984 |
|  |  | JP1259613C | 12.04.1985 |
| CN1246455A | 08.03.2000 | None |  |
| GB2366613A | 13.03.2002 | DE20013829U1 | 07.12.2000 |
|  |  | FR2813948A3 | 15.03.2002 |
| EP0140032A1 | 08.05.1985 | US4512863A | 23.04.1985 |
|  |  | JP60086265A | 15.05.1985 |
|  |  | JP6021345B | 23.03.1994 |
|  |  | JP1911090C | 09.03.1995 |
|  |  | US4563400A | 07.01.1986 |
|  |  | ES8601820A | 01.03.1986 |
|  |  | AU3235784A | 20.03.1986 |
|  |  | US4594137A | 10.06.1986 |
|  |  | CA1219548A | 24.03.1987 |
|  |  | US4692389A | 08.09.1987 |
|  |  | AU572881B | 19.05.1988 |
|  |  | CA1239614A | 26.07.1988 |
|  |  | EP0140032B | 28.02.1990 |
|  |  | DE3481433G | 05.04.1990 |
| JP2005002408A | 06.01.2005 | None |  |
| WO2004094685A2 | 04.11.2004 | US2005014010A1 | 20.01.2005 |

Form PCT/ISA/210 (patent family annex) (April 2007)

# INTERNATIONAL SEARCH REPORT

International application No.

PCT/CN2008/073544

CLASSIFICATION OF SUBJECT MATTER

C23C4/06 (2006.01) i

C23C4/04 (2006.01) i

Form PCT/ISA/210 (extra sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9964640 A1, Yokota Takeshi
- WO 9947721 A1, Tochihara Misako
- CN 1687200 A, Dehuan HUANG
- WO 9925898 A1, Shigeru Keijiro